# EUROPEAN PATENT APPLICATION

(11) **EP 3 456 322 A1**
(43) Date of publication of application: **20.03.2019**
(21) Application number: 16901559.1
(22) Date of filing: 11.05.2016
(51) Int. Cl.: A61K 9/48, A61J 3/07

(54) **SINGLE-DOSE CONTAINER AND METHOD FOR MANUFACTURING SAME**

(71) Applicant: Blanco Fernández, María Del Carmen, 43007 L'Arrabassada I La Savinosa (Tarragona) (ES)
(72) Inventor: Blanco Fernández, María Del Carmen, 43007 L'Arrabassada I La Savinosa (Tarragona) (ES)
(74) Representative: Herrero & Asociados, S.L.
(86) International application number: PCT/ES2016/070353
(87) International publication number: WO 2017/194794

(57) **Abstract**

The single-dose container comprises a casing (1) that houses a product (2) and opening means (3), characterised in that said casing (1) is formed from gelatine, water and glycerine.

The manufacturing method comprises the following steps: mixing gelatine, water and glycerine; forming a film of said mixture; moulding said film to form two halves of a casing (1); and injecting a product (2) and sealing said two halves of the casing (1) at the same time, housing said product (2) inside the casing (1).

A biodegradable container is obtained, which self-recycles to the extent it is manufactured with biodegradable products, and a leakproof container, since it is hermetically sealed by welding when manufactured.

## Description

The present invention relates to a single-dose container, which is manufactured only with biodegradable products. In addition, the present invention also relates to the manufacturing method of said single-dose container.

### Background of the invention

The use of single-dose containers is currently quite common, such that a predetermined quantity of a product, for example, oil, sauces, sugar, etc., is provided to users.

These single-dose containers are usually made of plastic and comprise opening means, such as a weakened area for tearing or separating the same from the rest of the container.

The single-dose containers made of plastic material that are currently known have the drawback that they are not biodegradable, which involves recycling costs after use.

Furthermore, these known single-dose containers also have the disadvantage that they are usually difficult to open, since their opening means require the user to have a certain skill due to the characteristics of the plastic material used for said container.

Therefore, the object of the present invention is to provide a single-dose container that is manufactured only from biodegradable materials, and that is also easy to open to deliver a precise serving where required, and a manufacturing method of a single-dose container of this type.

### Description of the invention

The single-dose container and the manufacturing method resolve the drawbacks mentioned above and exhibit other advantages that will be described below.

According to a first aspect, the present invention relates to a single-dose container comprising a casing that houses a product and opening means, which is characterised in that said casing is made of gelatine, water and glycerine.

Advantageously, said gelatine is a food-grade gelatine, and the thickness of said casing is between 0.60 and 0.80 mm.

According to a preferred embodiment, said opening means of the container comprise a protrusion defined on the casing, which can be broken, and can also comprise a weakened area, for example, a membrane, which breaks when pressure is applied to the casing.

If desired, said casing can also comprise colouring, opacifier and/or preservatives.

Preferably, in said casing the amount of gelatine is comprised between 35% and 45% by weight, the amount of water is comprised between 30% and 35% by weight, and the amount of glycerine is comprised between 25% and 35% by weight.

According to a preferred embodiment, said casing comprises 38% by weight of gelatine, 32% by weight of water and 30% by weight of glycerine.

According to a second aspect, the present invention relates to a manufacturing method of the single-dose container, characterised in that it comprises the following steps:
- mixing gelatine, water and glycerine;
- forming a film with said mixture;
- moulding said film to form two halves of a casing; and
- injecting a product and sealing said two halves of the casing at the same time, housing said product inside the casing.

Advantageously, the manufacturing method of the single-dose container according to the present invention comprises the steps of drying and curing the casing after injecting the product and sealing the two halves of the casing.

In addition, the mixing is preferably carried out by stirring the mixture at a temperature comprised between 65°C and 75°C, subsequently applying vacuum at a temperature between 75°C and 80°C, and subsequently cooling the mixture at a temperature comprised between 60°C and 70°C.

On the other hand, the film is preferably formed by expelling the mixture uniformly into a drum at a temperature between 18°C and 22°C, solidifying the mixture, and then moulding the film at a temperature comprised between 40°C and 50°C.

Advantageously, the two halves of the casing are vacuum sealed.

According to one embodiment, said drying process is carried out for a period of time of approximately 2 hours in continuous movement and using air, and said curing step is carried out for a period of time comprised between 20 and 24 hours.

The single-dose container and the method according to the present invention exhibits at least the following advantages:
- A biodegradable container is obtained, which self-recycles to the extent it is manufactured with biodegradable products.
- A leakproof container is obtained, since it is hermetically sealed by welding when manufactured;
- Unsealing or opening is easier than with conventional single-dose containers, since it is unsealed by exerting gentle pressure with the fingers on the protrusion and breaking it, without needing any additional accessories;
- It guarantees the preservation of the fundamental qualities of the product, since it is vacuum packed, which guarantees that the product housed inside will not oxidise;
- It makes the application process more precise, since the unsealing method makes it possible to apply the product and the quantity thereof in a controlled manner in the spaces where it is intended to be exclusively applied;
- It is more hygienic than conventional single-dose containers, as the inner product never comes into contact with the hands or with the outside of the container, which guarantees a hygienic application.

### Brief description of the drawings

To aid a better understanding of the above, drawings have been attached, in which a practical embodiment has been schematically represented and only by way of a non-limiting example.

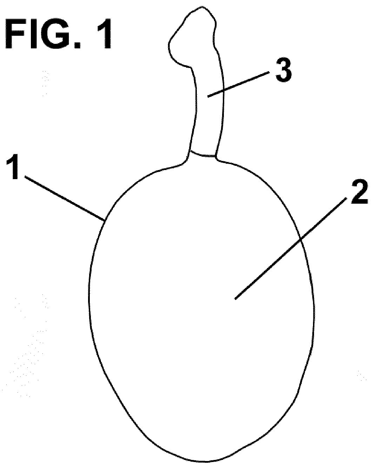

Figure 1 is a perspective view of the single-dose container according to the present invention.

### Description of a preferred embodiment

The single-dose container according to the present invention comprises a casing 1 inside which a product 2 is housed, for example, a liquid product such as olive oil, vinegar, milk or honey, or a sauce for use as dressing for food.

To open the container and supply said product, the single-dose container comprises opening means, such as a protrusion 3 defined in the casing 1, such that the protrusion 3 can be broken for the product 2 to come out. In addition, said protrusion 3 may comprise a weakened area, for example, a membrane (not shown in the figure), which breaks when pressing the casing. For example, said membrane may be placed at the end of the protrusion 3.

The casing 1 is formed only from biodegradable material, such as gelatine, water and glycerine, for example food-grade gelatine.

The thickness of said casing 1 is sufficient to securely contain said product 2 and allow it to be served once the container is opened, for example, between 0.60 mm and 0.80 mm.

According to a second aspect, the present invention relates to the manufacturing method of said single-dose container.

First of all, in this method, the material that will form the casing 1 is manufactured.

In particular, gelatine, water and glycerine are mixed first by being introduced into a tank and stirred at a temperature of, for example, 70°C. Once the material is melted and mixed, the temperature is increased to 78°C to perform a vacuum process and extract all the air. Once all the air has been extracted, the temperature is lowered to 65°C.

This mixture, which is used to manufacture the casing 1, may comprise, for example:
Gelatine: 38% by weight;
Water: 32% by weight; and
Glycerine: 30% by weight.

In some cases, the following ingredients can be added to this material to obtain special characteristics such as colour, brightness, opacity, etc. For example, food colouring, an opacifier, preservatives, etc. may be added.

For the product 2 that will be placed inside the casing 1, first the product is provided to a hopper of the machine where the container is manufactured, and once the material of the casing 1 has been manufactured and we have the product in the machine, the process of packaging the product 2 begins.

To achieve this, the material of the casing 1 is introduced into a steel compartment at a temperature of 65°C. In this compartment the material can be supplied uniformly on a drum cooled to 20°C, which causes the material to solidify and creates a film of about 0.60 mm in thickness.

Next, the film passes through moulds with the desired shape of the container.

Just at the moment in which the material passes through the mould, it is heated by a steel wedge at a temperature between 40°C and 50°C, depending on the room temperature, forming two casing halves 1.

This contribution of heat allows the fusion and welding of the two halves that will make up the final casing 1. Before the welding and fusion process, and in tenths of a second, the material, for example, olive oil, is injected, and immediately afterwards the casing 1 is hermetically and vacuum-sealed.

Then, the single-dose container (the casing 1 with the product inside) is placed in drying drums, which are moved by a conveyor belt where they will dehydrate for a period of time of about 2 hours. These drying drums are kept in continuous rotary motion with air supplied through fans.

After two hours of dehydration, the containers are removed from the drums and passed on a tray to a curing room, where they remain for about 22 hours. After that time, they leave the curing room and the single-dose containers are ready for subsequent packaging, labelling and storage to be later sold.

Although reference has been made to several specific embodiments of the invention, it is obvious to a person skilled in the art that the single-dose container described is subject to numerous variations and modifications, and that all the details mentioned can be replaced by other technically equivalent ones, without departing from the scope of protection defined by the claims attached hereto.

## Claims

1. A single-dose container, comprising a casing (1) that houses a product (2) and opening means (3), **characterised in that** said casing (1) is formed from gelatine, water and glycerine.

2. The single-dose container according to claim 1, wherein said gelatine is food-grade gelatine.

3. The single-dose container according to claim 1, wherein the thickness of said casing (1) is comprised between 0.60 and 0.80 mm.

4. The single-dose container according to claim 1, wherein said opening means comprise a breakable protrusion (3) defined in the casing (1).

5. The single-dose container according to claim 1, wherein said casing (1) also comprises colourings, opacifiers and/or preservatives.

6. The single-dose container according to claim 1, wherein in said casing (1) the amount of gelatine is comprised between 35% and 45% by weight, the amount of water is comprised between 30% and 35% by weight, and the amount of glycerine is comprised between 25% and 35% by weight.

7. The single-dose container according to claim 6, wherein said casing (1) comprises 38% by weight of gelatine, 32% by weight of water and 30% by weight of glycerine.

8. A manufacturing method of the single-dose container, **characterised in that** it comprises the following steps:
- mixing gelatine, water and glycerine;
- forming a film with said mixture;
- moulding said film to form two halves of a casing; and
- injecting a product (2) and sealing said two halves of the casing (1) at the same time, housing said product (2) inside the casing (1).

9. The manufacturing method of the single-dose container according to claim 8, which comprises the steps of drying and curing the casing (1) after injecting the product (2) and sealing the two halves of the casing (1).

10. The manufacturing method of the single-dose container according to claim 8, wherein said mixing is preferably carried out by stirring the mixture at a temperature comprised between 65°C and 75°C, subsequently applying vacuum at a temperature between 75°C and 80°C, and subsequently cooling the mixture at a temperature comprised between 60°C and 70°C.

11. The manufacturing method of the single-dose container according to claim 8, wherein the film is formed by expelling the mixture uniformly into a drum at a temperature comprised between 18°C and 22°C, solidifying said mixture.

12. The manufacturing method of the single-dose container according to claim 8, wherein the film is moulded at a temperature comprised between 40°C and 50°C.

13. The manufacturing method of the single-dose container according to claim 8, wherein the two halves of the casing (1) are vacuum sealed.

14. The manufacturing method of the single-dose container according to claim 9, wherein said drying is carried out for a period of time of approximately 2 hours in continuous movement and using air.

15. The manufacturing method of the single-dose container according to claim 9, wherein the curing step is carried out for a period of time comprised between 20 and 24 hours.
